# EUROPÄISCHE PATENTSCHRIFT

(11) **EP 2 888 779 B1**
(45) Veröffentlichungstag und Bekanntmachung des Hinweises auf die Patenterteilung: **02.10.2019**
(21) Anmeldenummer: 13762373.2
(22) Anmeldetag: 23.08.2013
(51) Int. Cl.: H01M 4/66, H01M 4/72, H01M 4/74, H01M 4/88, H01M 8/0245, A61B 5/00, A61N 1/04, D05C 17/00, H01G 11/24

(54) **ELEKTRODE FÜR GALVANISCHE ZELLE**
ELECTRODE FOR A GALVANIC CELL
ÉLECTRODE POUR CELLULE GALVANIQUE

(30) Priorität: 24.08.2012 AT 503432012
(43) Veröffentlichungstag der Anmeldung: 01.07.2015
(73) Patentinhaber: Universität Innsbruck, 6020 Innsbruck (AT); V-Trion GmbH, 6845 Hohenems (AT); "Riedmann Markus" GmbH, 6890 Lustenau (AT); HKG-Embroideries Hofer GmbH, 6890 Lustenau (AT)
(72) Erfinder: FROEIS, Thomas, A-6833 Weiler (AT); LENNINGER, Margit, A-6863 Egg (AT); BECHTOLD, Thomas, A-6850 Dornbirn (AT); GRABHER, Günter, 6911 Eichenberg (AT); HOFER, Jutta, A-6890 Lustenau (AT); RIEDMANN, Markus, A-6973 Höchst (AT)
(74) Vertreter: Schwarz & Partner Patentanwälte OG
(86) Internationale Anmeldenummer: PCT/AT2013/050165
(87) Internationale Veröffentlichungsnummer: WO 2014/028958

(56) Entgegenhaltungen:
- WO-A1-2008/022482
- DE-B1- 1 596 216
- US-A- 4 134 192
- US-A1- 2009 130 549
- US-A1- 2009 311 587
- MARGIT LENNINGER ET AL: "High current density 3D electrodes manufactured by technical embroidery", JOURNAL OF SOLID STATE ELECTROCHEMISTRY, Bd. 17, Nr. 8, 9. Mai 2013 (2013-05-09), Seiten 2303-2309, XP055094442, ISSN: 1432-8488, DOI: 10.1007/s10008-013-2108-1
- BEN BREITUNG ET AL: "Embroidered Copper Microwire Current Collector for Improved Cycling Performance of Silicon Anodes in Lithium-Ion Batteries", SCIENTIFIC REPORTS, vol. 7, no. 1, 1 December 2017 (2017-12-01), XP055573426, DOI: 10.1038/s41598-017-13261-y
- JAN MEYER ET AL: "Textile Pressure Sensor for Muscle Activity and Motion Detection", WEARABLE COMPUTERS, 2006 10TH IEEE INTERNATIONAL SYMPOSIUM ON, 1 October 2006 (2006-10-01), pages 69-72, XP055573649, Pi DOI: 10.1109/ISWC.2006.286346 ISBN: 978-1-4244-0597-8

## Beschreibung

Die Erfindung betrifft eine Elektrode für eine elektrochemische Zelle mit einer Oberflächenstruktur sowie eine galvanische Zelle und ein Halbelement, umfassend eine Elektrode.

Energieerzeugung und Energiespeicherung spielen bei mobilen Anwendungen eine zentrale Rolle. Um dem zunehmenden Bedarf mobiler Energieerzeugung und Energiespeicherung Rechnung zu tragen, steht die Entwicklung elektrochemischer Verfahrenstechniken im Vordergrund. Die mobile Energieerzeugung und Energiespeicherung wird zum allergrößten Teil über elektrochemische bzw. galvanische Zellen bewerkstelligt. Unter einer elektrochemischen oder galvanischen Zelle versteht man einen Energiespeicher und Energiewandler, der chemische Energie (Reaktionsenthalpie) in elektrische Energie umwandelt und *vice versa.* Hierzu gehören beispielsweise Batterien, Akkumulatoren, Superkondensatoren und Brennstoffzellen. Allen diesen Energiespeichersystemen gemeinsam ist die wechselweise Überführung elektrischer Energie in energiereiche chemische Stoffe und umgekehrt. Diese Energieumwandlung erfolgt je nach Konstruktion der elektrochemischen Zelle und Effektivität des Systems mehr oder weniger vollständig.

Bei der Entwicklung und Dimensionierung von elektrochemischen Energiespeichern gibt es unterschiedliche Kenngrößen, wobei diese Kenngrößen in weiterer Folge auch die Einsatzmöglichkeiten des elektrochemischen Energiespeichers festlegen. Folgende wichtige Kenngrößen sind zu berücksichtigen:
- Die Energieeffizienz beschreibt, wieviel der beim Aufladen des elektrochemischen Energiespeichers eingesetzten elektrischen Energie beim Entladen wieder gewonnen wird.
- Die Coulometrische Effizienz beschreibt, welche Ladungsmenge effektiv in chemische Speicherstoffe reversibel umgewandelt wird.
- Die Energiespeicherkapazität (kWh) beschreibt die durch chemische Umwandlung maximal im elektrochemischen Energiespeicher speicherbare Energie, welche durch die in einem Akkumulator/Batterie vorhandenen Mengen an Chemikalien und die Zellenspannung des verwendeten Systems definiert wird.
- Die tatsächlich nutzbare Energie wird durch den unter Arbeitsbedingungen tatsächlich erreichbaren Grad der chemischen Umwandlung bei Aufladung/Entladung vorgegeben. Sie liegt normal deutlich unter dem theoretisch berechenbaren Wert. In der Praxis bedeutet dies, dass bei vielen Batterien und Akkumulatoren die energetische Ausnutzung der vorhandenen Chemikalien nur unvollständig ist.
- Die Energiedichte (Wh/kg) beschreibt die pro kg Zellengewicht speicherbare Energie. Dieser Wert liegt bei Batterien/Akkumulatoren mit beispielsweise rund 200 Wh/kg relativ hoch, liegt jedoch deutlich unter den theoretisch berechneten Werten (Claus Daniel und Jürgen O. Besenhard, Handbook of Battery Materials, Vol 1 und 2, Wiley- VCH, 2011, ISBN 978-3-527-32695-2).
- Die Leistungsdichte (W/kg) gibt die maximale abgebbare elektrische Leistung pro kg Zellengewicht an. Typische Werte liegen bei Batterien/Akkumulatoren mit 10 bis 100 W/kg deutlich unter den Leistungen der Superkondensatoren, welche zwischen 10³ bis 10⁶ W/kg speichern können. Allerdings erreichen solche Superkondensatoren nur eine sehr geringe Energiedichte von 0.01 - 0.1 Wh/kg (T. Nguyen, R.F. Savinell, Flow Batteries, The Electrochemical Society Interface, Fall 2010, 54-55).

Die außerordentlich hohe Leistungsdichte der Superkondensatoren ergibt sich aus der Tatsache, dass die elektrochemische Ladungsspeicherung nur an einer sehr dünnen Grenzfläche zum Elektrolyt erfolgt und damit eine sehr rasche Aufladung/Entladung erlaubt. Die geringe Dicke der Schicht bedingt damit auch die geringe Energiedichte, welche den Einsatz zur längeren Energiespeicherung verhindert.

Die höhere Energiedichte der Akkumulatoren und Batterien wird durch die Verwendung entsprechend großer Mengen an aktiven Massen erreicht. Um eine elektrochemische Umsetzung zu erreichen, müssen die aktiven Massen poröse Strukturen aufweisen und gleichzeitig in elektrischen Kontakt mit der Ableitelektrode gebracht werden. Eine dickere aktive Schicht erhöht die Speicherkapazität einer Konstruktion, jedoch ist durch die poröse Struktur die elektrische Leitfähigkeit begrenzt, was mehrere Nachteile mit sich bringt: Die Zellen haben einen relativ hohen inneren Widerstand, d.h. bei hohen Stromdichten sinkt die Zellenspannung und damit der Wirkungsgrad stärker. Die Ausnutzung der aktiven Masse erfolgt nur teilweise, sodass die effektiv nutzbare Energiedichte verringert wird. Die Aufladung des Akkumulators wird durch die maximale einbringbare Stromdichte begrenzt, welche wiederum vom Stromübergang von der Ableitelektrode zur aktiven Masse begrenzt wird.

Die begrenzte Leitfähigkeit der verschiedenen aktiven Massen hat zur Folge, dass viele Zellenkonzepte nur dünne Schichten verwenden, in der Folge aber hohe Flächen bei den Ableitelektroden aufweisen müssen und damit zusätzliches unerwünschtes Gewicht erhalten. Bei vielen Akkumulatorkonzepten führen die chemischen Umwandlungen während der Aufladung/Entladung auch zu Dimensionsveränderungen in der aktiven Elektrodenmasse, was die mechanische Empfindlichkeit erhöht und für die Stromübertragung nachteilig ist. Abgelöste Teile der Elektrodenfüllmasse können z.B. bei Bleiakkumulatoren zum inneren Kurzschluss und Ausfall des Elements führen.

Eine Möglichkeit, um die Vorteile der höheren Energiedichte der Batterien/Akkumulatoren mit der höheren Leistungsdichte der Superkondensatoren zu verbinden, ist die Verwendung von dreidimensionalen Elektrodenstrukturen, welche die Stromzufuhr und Stromabfuhr in die Tiefe der aktiven Masse verbessern. Nach dem Stand der Technik sind verschiedenste Konzepte zur Integration leitender Strukturen in elektrochemisch aktive Massen von Akkumulatoren/Batterien beschrieben worden.

Die US 4,250,235 offenbart ein durch Ätzen hergestelltes Nickelmetallgitter als leitenden Support für die aktive Masse in einer NiH-Batterie. In der US 3,441,390 wird die Verwendung eines Nickel-Pulvers und von Nickel-Metallfasern zur Herstellung einer Elektrode gezeigt. Die US 3,960,601 offenbart die Verwendung von Carbonfasern zur Herstellung einer leitenden Elektrodenstruktur. Nach der US 4,731,311 wird eine leitende poröse Schwammstruktur durch Polymerisation entsprechender Ausgangsmaterialien als Elektrodenbeschichtung erzeugt. Die Verwendung von Streckmetallkonstruktionen in Elektrolysezellen wurde in der DE 10 2004 023 161 A1 gezeigt.

Die im Stand der Technik beschriebenen Konzepte erhöhen die Leitfähigkeit in der porösen Struktur. Die Stromzufuhr erfolgt jedoch stets von der Rückseite der elektrischen Zelle, was je nach Porosität der Masse, Leitfähigkeit der stromleitenden Materialien und der Elektrolytleitfähigkeit wiederum die insgesamt erreichbare Stromdichte bzw. den Zellenstrom begrenzt. Die nach dem Stand der Technik vorgeschlagenen Lösungen verbessern daher das Verhalten in verhältnismäßig dünnen Schichten, sind jedoch von begrenztem Wert, sobald die aktive Masse eine Dicke von einigen mm erreicht.

Auch die Verwendung von textilen Materialien (Vliese, Gewebe) als Stromteiler wurde in der Literatur beschrieben. In der US 5,242,768 werden dreidimensionale Gewebe für die Herstellung leitender Elektrodenstrukturen beschrieben. Nach der DE 195 06 496 und der DE 197 20 792 werden dreidimensionale gitterartige Metallstrukturen als Elektroden durch nichtleitende Elemente voneinander getrennt. Nachteile dieser Konzepte ergeben sich aus mehreren Bereichen: Die Materialien sind auf die Zellendimension durch Zuschnitt anzupassen, was Materialabfälle, erhöhte Kosten und technische Problem an den Schnittkanten verursacht.

Die Kontaktierung der Materialien und der elektrische Übergang zur Ableitelektrode sowie die Gewährleistung einer einheitlichen Stromverteilung verursachen zusätzlichen Aufwand. In einer bekannten Ausgestaltung von Elektrolysezellen mit dreidimensionalen Elektroden für Elektrolyte mit niederen Stromdichten nach der WO 95/07374 A1 werden mehrere Elektrodenelemente aus Edelstahlgewebe separat mit Strom versorgt und im selben Elektrolyt zu einer gemeinsame Elektrode zusammengefasst. Solche Mehrkathoden bzw. Mehrelektrodenzellen erreichen wesentlich höhere Elektrodenflächen im Vergleich zu Plattenkonstruktionen. Der hohe technische Aufwand zur Herstellung solche Mehrelektrodenkonstruktionen hat deren Einsatz auf spezielle Anwendungsfälle begrenzt.

DE 15 96 216 B1 offenbart eine Elektrode für eine galvanische Zelle, in welcher ein Graphitfaserfilz und ein metallisches Gewebe mit einem Nickeldraht vernäht sind.

US 4,134,192 beschreibt eine Elektrode für eine galvanische Zelle, umfassend ein Gewebe in welches leitende Fäden eingenäht wurden. Das Gewebe kann z.B. Glasfaser oder Kunststoff sein.

In US 2009/130549 A1 wird eine Elektrode für eine galvanische Zelle mit einem Träger, welcher mit einer geschäumten Carbonschicht vernäht wurde, beschrieben.

US 2009/311587 A1 offenbart Elektroden aus nichtleitendem Gewebe mit eingewebten, zweidimensionalen Metallstrukturen.

Es ist daher Aufgabe der gegenständlichen Erfindung eine galvanische Zelle bereit zu stellen, bei der die genannten Probleme verringert sind. Insbesondere soll die galvanische Zelle eine optimierte Stromverteilung gewährleisten bei gleichzeitig hoher Energiedichte und hoher Leistungsdichte. Insbesondere soll ermöglicht werden, dass die aktive Masse eine Dicke von einigen mm aufweisen kann.

Diese Aufgabe wird gelöst durch eine Elektrode für eine galvanische Zelle, gekennzeichnet durch einen elektrisch isolierenden Träger, welcher mit einer Stickerei versehen ist, wobei zumindest ein Bereich der Oberfläche der Stickerei elektrisch leitend ausgebildet ist, wobei die Stickerei eine dreidimensionale elektrisch leitende Struktur bildet und Fäden der Stickerei in Stickzahl und/oder der Stickdichte variiert sind und/oder unterschiedlich in der Elektrodentiefe verteilt sind, wobei Teile der Stickerei als Luftstickerei ausgebildet sind.

Vereinfacht ausgedrückt handelt es sich um eine gestickte Elektrode für elektrochemische Prozesse, bei der ein elektrisch leitendes Material auf ein Trägermaterial so durch Stickereitechnik befestigt wird, dass eine dreidimensionale elektrisch leitende Struktur gebildet wird. Auf diese Art und Weise kann die Elektrode in einer galvanischen Zelle eine aktive Masse mit mehreren mm Dicke aufweisen, wobei eine optimierte Stromverteilung und eine hohe Energiedichte sowie Leistungsdichte möglich sind.

Demzufolge wird die Aufgabe auch durch eine galvanische Zelle, umfassend zwei Elektroden, die jeweils in einen Elektrolyten eingebettet sind, gelöst, wobei zumindest eine Elektrode einen elektrisch isolierenden Träger, welcher mit einer Stickerei versehen ist, wobei zumindest ein Bereich der Oberfläche der Stickerei elektrisch leitend ausgebildet ist, wobei die Stickerei eine dreidimensionale elektrisch leitende Struktur bildet und Fäden der Stickerei in Stickzahl und/oder der Stickdichte variiert sind und/oder unterschiedlich in der Elektrodentiefe verteilt sind, wobei Teile der Stickerei als Luftstickerei ausgebildet sind. Weiters wird die Aufgabe durch ein Halbelement umfassend eine solche Elektrode gelöst.

Nachfolgend werden Ausführungen und vorteilhafte Varianten beschrieben, die gleichermaßen für die Elektrode allein als auch für die galvanische Zelle und das Halbelement gelten.

Hinsichtlich der Struktur kann beispielhaft genannt werden, dass der Träger ein Vlies, eine Folie, ein Gewebe, ein Gitter oder eine Mischung daraus aufweist.

In einer Ausführungsvariante ist vorgesehen, dass der Träger und die Stickerei mit einer elektrisch leitenden Beschichtung versehen sind, welche nach dem Aufbringen der Stickerei auf den Träger aufgebracht wurde. In diesem Fall können sowohl Stickerei als auch Träger an der Oberfläche elektrisch isolierend ausgebildet sein und erst durch die Beschichtung zu leitenden Strukturen werden.

Das Grundgerüst ist aus einem elektrisch isolierenden Material aufgebaut. Hierfür können exemplarisch Kunststoffe, Naturstoffe, Textile usw. genannt werden. Als Grundmaterial käme für einen nicht leitenden Träger auch ein PVA-Vlies, Celluloseacetat, Cellulose oder eine Mischung daraus in Frage. Es ist vorgesehen, dass Teile des Trägers nach dem Aufbringen der Stickerei wieder entfernt werden, beispielsweise durch Abtragen, Auflösen, Sandstrahlen etc. Man spricht in solchen Fällen auch von einer Luftstickerei. So kann z.B. ein Bauwollgewebe durch Säurebehandlung, Celluloseacetat durch Auflösung in Aceton oder Polyvinylakoholvlies durch Auflösung in heißem Wasser entfernt werden.

In der einfachsten Ausführungsvariante ist vorgesehen, dass die Stickerei aus einem zumindest an der Oberfläche elektrisch leitenden Material besteht. In diesem Fall kann also ein elektrischer Leiter (z.B. elektrischer Draht) in den Träger eingestickt wird.

Andere Varianten sehen vor, dass die Stickerei an der Oberfläche elektrisch isolierend ausgebildet ist und erst durch nachträgliche Beschichtung leitend gemacht wird. In Ausführungsvarianten kann vorgesehen sein, dass die Stickerei aus Metallfasern, Folienstreifen, Drähten, Carbonfasern, Fasern - vorzugsweise Kunststofffasern oder Spinnfasern - welche gegebenenfalls eine elektrisch leitende Beschichtung aufweisen, oder einer Kombination daraus besteht.

Der Einfachheit halber wird das Basismaterial, aus dem die Stickerei besteht, nachfolgend als Faden bezeichnet, unabhängig davon, ob es sich um eine Faser, einen Draht, eine Schnur, ein Band usw. handelt. Als elektrisch leitende Fadenmaterialien können Spinnfäden aus leitenden Materialien und leitende beschichtete Fäden genannt werden. In einer bevorzugten Form werden Metallfäden zur Bildung von leitenden Strukturen verwendet, in einer besonders bevorzugten Form werden Fäden aus Carbonfasern, Al, Cu, Ag, Ni, Cr, Pt, Fe und deren Legierungen, z.B. Edelstahl, verwendet. Besonders gut eignen sich Materialien als Drähte bzw. Spinnfasern, die in einer herkömmlichen Stickerei verarbeitbar sind.

In einer bevorzugten Ausführungsvariante ist vorgesehen, dass der Faden, aus dem die Stickerei ausgeführt ist, auch eine Anschlussfahne für einen Leiter aufweist. Das bedeutet, dass auf ein Anlöten einer Anschlussfahne an den Faden verzichtet werden kann. Der Faden wird in einem Bereich - insbesondere der Stickerei - einfach derart gefaltet und gestaltet, dass eine Anschlussfahne gebildet wird, an der ein Leiter für die restliche galvanische Zelle angeschlossen wird.

In einer weiteren Ausführungsvariante kann vorgesehen sein, dass die Leiterdichte an der Oberfläche der Elektrode über die Elektrodenlänge und -breite sowie über die Elektrodentiefe derart verteilt ist, dass im Betriebszustand eine homogene Stromdichtenverteilung erzielbar ist. Durch das Sticken wird die Elektrodenfläche so angepasst, dass die Stromdichteverteilung optimiert wird, beispielsweise durch Variation der Stickzahl und/oder der Stickdichte und/oder durch unterschiedliche Verteilung der Fäden in der Elektrodentiefe, sodass eine gezielte Vergleichmäßigung der Stromzufuhr erreicht wird. Die (gleichmäßige) Stromdichteverteilung kann über die elektrochemisch erzielte Umsetzung an der Elektrodenseite überprüft werden. Gegebenenfalls kann dies durch gezielte Messung der Stromdichteverteilung in der Elektrode selbst bestätigt werden.

Grundsätzlich sind die erfindungsgemäßen Elektroden in galvanischen Zellen aller Art einsetzbar. Es kommen dabei neben Primärzellen (z.B. Batterien), Sekundärzellen (z.B. Akkumulatoren) auch Brennstoffzellen in Frage.

Dementsprechend ist in einem Aspekt der Erfindung vorgesehen, dass es sich bei der galvanischen Zelle um eine Primärzelle, eine Sekundärzelle oder eine Brennstoffzelle handelt.

Für eine Brennstoffzelle hat es sich als vorteilhaft erwiesen, wenn zumindest ein katalytisch wirksames Material auf der elektrisch leitenden Oberfläche, vorzugsweise durch Soutache-Technik aufgebraucht ist.

In einem Aspekt der Erfindung betrifft diese auch ein Verfahren zur Herstellung einer Elektrode und einer galvanischen Zelle. Aus den oben genannten Eigenschaftsprofilen lassen sich die entsprechenden Verfahrensschritte ableiten.

Weitere Details und Vorteile der Erfindung sowie Ausführungsvarianten werden nachfolgend erläutert.
- Die Fig. 1: zeigt eine schematische Darstellung einer erfindungsgemäßen Elektrode.
- Die Fig. 2 bis 4: zeigen drei Ausführungsvarianten für erfindungsgemäße Elektroden.

Für die Optimierung der Leistungsfähigkeit einer Batterie ist eine optimierte Stromverteilung in der aktiven Masse erforderlich, um eine optimale Ausnutzung der aktiven Masse bei hoher Stromdichte zu ermöglichen.

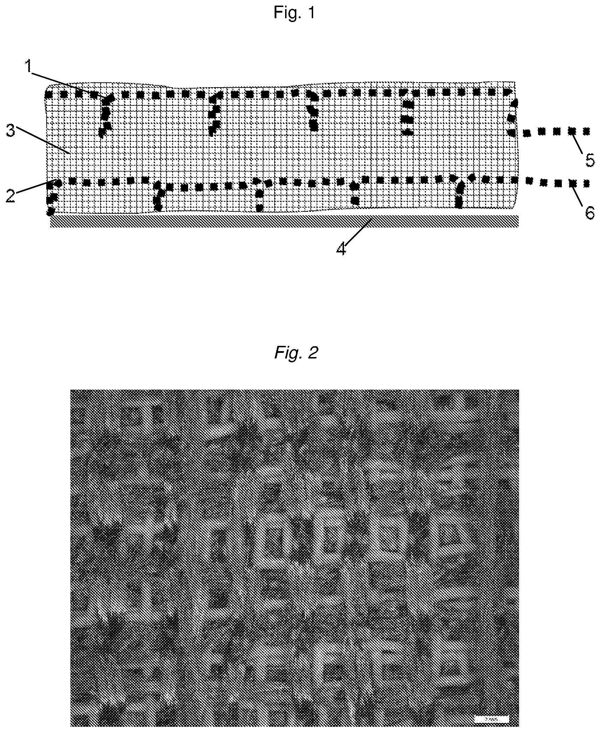

Fig. 1 zeigt schematisiert ein Ausführungsbeispiel der Erfindung im Querschnitt. Erkennbar ist der Träger 3, der ein poröses textiles Grundmaterial darstellt und eine Matrix für die Stickerei bildet. Im gegenständlichen Fall sind zwei Stickereien 1, 2 erkennbar, welche aus einem leitenden Material in den Träger 3 eingestickt wurden. Das gezeigte Ausführungsbeispiel zeigt, dass jede der beiden elektrisch leitenden Stickereien jeweils eine Ableitelektrode 5, 6 aufweisen. Im vorliegenden Bild sind die beiden Stickereien 1, 2 getrennt geführt, daher ist hier eine Ausführung als Mehrkathodenzelle dargestellt. Damit handelt es sich um eine Mehrfachelektrode. In einer anderen Ausführungsform werden die beiden Ableitelektroden 5 und 6 gemeinsam geführt und sind elektrisch verbunden, sodass es eine sich um eine einfache Zelle handeln würde. Die Ableitelektroden 5, 6 können auch als Anschlussfahne für einen Leiter ausgebildet sein. In einer galvanischen Zelle befindet ist die obere Seite der Gegenelektrode zugewandt. An der Unterseite der Elektrode befindet sich die Rückwand 4 der elektrochemischen Zelle.

Auf einem Träger werden Fäden aus leitendem oder nichtleitendem Material durch Stickerei so befestigt, dass eine dreidimensionale elektrisch leitende Struktur gebildet wird, welche als Elektrode für elektrochemische Anwendungen bzw. Stromverteiler bzw. Fäden oder Drähte geeignet sind. Überraschenderweise eignet sich die Technik der Stickerei außerordentlich gut zur Herstellung leitender dreidimensionaler Elektrodenstrukturen für elektrochemische Anwendungen. Durch Anwendung der technischen Stickerei lassen sich leitende Strukturen als Stromverteiler für elektrochemische Verfahren erzeugen.

Zur Erzeugung der dreidimensionalen Elektrodenstruktur werden leitende Materialien und nichtleitende Elemente so miteinander kombiniert, dass die geforderte Dichte an stromverteilenden Leiterbahnen in Elektrodenbereich erreicht wird. In einer besonders vorteilhaften Ausführung wird eine Elektrode als Motiv aus einem leitenden Faden so gestickt, dass keine leitenden offenen Fadenenden im aktiven Teil der Elektrode verbleiben, sodass ein wesentlich geringeres Risiko für innere Kurzschlüsse gegeben ist. Die leitenden Elemente der Elektrodenstruktur können aus der Elektrode herausgeführt werden und damit auch gleichzeitig zur Kontaktierung eingesetzt werden. In einer besonderen Ausführungsform verändert sich die Dichte an stromverteilenden Leiterelementen über die Länge der Elektrode mit zunehmender Entfernung von der Kontaktierung um eine homogene Stromverteilung über die Elektrodenlänge zu gewährleisten.

Die gestickte Elektrodenstruktur übernimmt die Funktion der Stromverteilung in der aktiven Masse des galvanischen Elements. Die technische Vorgabe der Auslegung von Elektrodenmaterial, Leiterdichte und Dicke der 3-D Struktur hängt von der verwendeten elektrochemisch aktiven Füllmasse ab und wird nach den bekannten Vorgaben für dreidimensionale poröse Elektroden berechnet. Die Vorteile der Verwendung einer gestickten Elektrodenstruktur mit hoher Elektrodenoberfläche lassen sich in Messanordnungen zur Bestimmung der bei gegebenem Elektrodenpotential erreichbaren Stromdichte direkt feststellen. Die Stromdichte bezeichnet den - bezogen auf eine bestimmte Elektrodenfläche - erreichbaren Strom. Üblicherweise wird die geometrische Fläche (projizierte Oberfläche; Oberflächeninhalt bezogen auf die Dimension) der Elektrode für die Berechnung der Stromdichte als Bezugswert herangezogen, da die effektive (innere; tatsächliche) Oberfläche des Materials insbesondere bei dreidimensionalen Elektroden nicht ohne weiteres definiert werden kann. Elektroden mit hoher innerer Oberfläche (z.B. dreidimensionale Elektroden, Schüttelektroden, Vliese, Gewebeelektroden oder gestickte Elektroden) weisen daher in Messanordnungen zur Bestimmung der Stromdichte durch ihre höhere innere Oberfläche, bezogen auf die geometrische Oberfläche entsprechend höhere Stromdichten auf.

In den Anwendungsbeispielen werden Voltammogramme an planen Metall- Elektroden mit Voltammogrammen an gestickten Elektroden verglichen. Dazu strömt die Lösung eines reversiblen Redoxsystems an einer Elektrode vorbei, deren Potential gegenüber einer Referenzelektrode stufenweise zu negativen Werten gesteigert wird. Erreicht das Elektrodenpotential die Höhe des Redoxpotentials des reversiblen Redoxpaars, so beginnt ein für die Konzentration des Redoxpaars charakteristischer Strom zu fließen. Unter diffusionskontrollierten Bedingungen hängt dabei die maximal erreichbare Höhe der Stromdichte von der Konzentration des Redoxpaars ab. Während bei einer planen Metallelektrode die geometrische Oberfläche die erreichbare maximale Stromdichte bei gegebenen Elektrodenpotential definiert, sind bei dreidimensionalen Elektroden entsprechend höhere Werte zu erwarten. Aus der unter vergleichbaren experimentellen Bedingungen erreichten Stromdichte kann daher die Leistungsfähigkeit einer Elektrodenkonstruktion abgeleitet werden.

### Anwendungsbeispiele:

Für die Anwendungsbeispiele wurden verschiedene Elektrodenvarianten gewählt.

### Elektrodenvariante A: (Fig. 2)

Aufbau der Kathode: Edelstahl-Draht (Nm 22/2 Stahlfaserzwirn, DIN 14401) im Abstand von 2 mm auf Edelstahlvlies gestickt, eine weitere Stickreihe zwischen den Stickreihen im Abstand von 1 mm versetzt und durch einen PES-Gitter- Distanzgewebe 1 mm nach vorne versetzt aufgestickt. Das Material wurde auf einem mit Cu/Ni 3015 beschichteten Polyestervlies als Grundmaterial hergestellt.

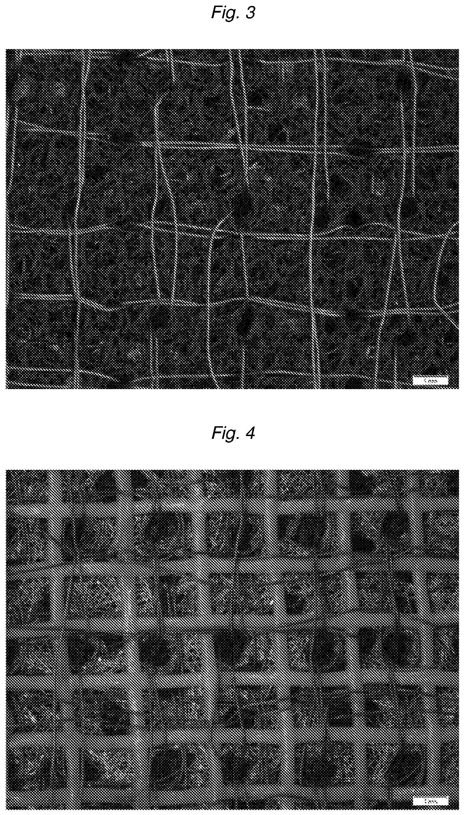

### Elektrodenvariante B: (Fig. 3)

Elektrodenvariante B ist analog aufgebaut wie Elektrodenvariante A, allerdings wurde Cu-Draht anstelle des Edelstahldrahts verwendet

### Elektrodenvariante C: (Fig. 4)

Elektrodenvariante C ist analog aufgebaut wie Elektrodenvariante B, allerdings wurde Cu-Draht in höherer Stickdichte verwendet.

### Anwendungsbeispiel 1:

Die Experimente wurden bei Raumtemperatur durchgeführt. Die Voltammogramme wurden in einer Durchflusszelle mit paralleler Geometrie durchgeführt. Katolyt und Anolyt werden durch eine Kationtauschermembrane getrennt (Nafion Type). Als Kathode wird zu Vergleichszwecken eine plane Cu-Kathode mit 100 cm² Fläche verwendet (Cu-Folie, Merck, Darmstadt, Deutschland), für die Anwendungsbeispiele, die durch Stickerei hergestellte dreidimensionale Elektrode mit derselben Fläche (Elektrodenvarianten A bis C, siehe oben). Die Anode besteht aus Edelstahl.

Die Versuchslösung wird als Katolyt verwendet und wird mithilfe einer Schlauchpumpe durch den Kathodenraum gepumpt (Strömungsgeschwindigkeit 150 ml/min). Dies entspricht einer Katolytströmung von rund 0,15 cm s⁻¹. Das Gesamtvolumen der Katolytlösung betrug 800 ml. 400 ml 1 M NaOH wurden zur Füllung des Anodenraums verwendet. Im Katolyt wurde 0,1 M NaOH als Grundelektrolyt verwendet. Die Zellenspannung wird entweder manuell durch Regelung an einer Laborstromversorgung eingestellt oder durch einen Potentiostaten so gesteuert, dass sich ein erwünschtes Kathodenpotential einstellt. Das Kathodenpotential wird gegenüber einer (Ag/AgCl, 3M KCl) Referenzelektrode gemessen. Bei manueller Regelung wird ein Potentiometer verwendet (Metrohm pH meter 654, Fa. Metrohm, Herisau, Switzerland) bei Verwendung des Potentiostaten übernimmt dieser die Potentialmessung und Regelung. Durch Messung des Zellenstroms als Funktion des sich schrittweise verändernden Kathodenpotentials wird ein Voltammogram vermessen.

Eine detaillierte Beschreibung der Zellenanordnung findet sich in der Literatur (Bechtold T., Turcanu A., Schrott W., "Electrochemical reduction of CI Sulfur Black 1 - Correlation between electrochemical parameters and colour depth in exhaust dyeing", J. Appl. Electrochem., 38 (2008) 25-30; A. Turcanu, T. Bechtold, Indirect cathodic reduction of dispersed indigo by 1,2-dihydroxy-9,10-anthraquinone-3-sulphonate (Alizarin Red S), Journal of Solid State Electrochemistry, 15/9, (2011) 1875-1884. Turcanu A., Fitz-Binder C, Bechtold T., Indirect cathodic reduction of dispersed CI Vat Blue 1 (indigo) by dihydroxy-9,10-anthraquinones in cyclic voltammetry experiments, Journal of Electroanalytical Chemistry, 654/1-2, (2011) 29-37). Als elektrochemisch reversibles Redoxsystem wird eine 0.005 mol/l Anthrachinon-1,5-disulfonsäure Lösung verwendet. Die Voltammogramme werden bei unterschiedlichen Spannungsvorschubgeschwindigkeiten v aufgezeichnet. Bei beiden Elektrodentypen wird die Zellenstromstärke bei einem Katodenpotential von -800 mV und - 900 mV in Tabelle 1 dargestellt.

**Tabelle 1. (GE = Grundelektrolyt)**

| Katodenpotential | *v* | Stromdichte | | | | | | | |
|---|---|---|---|---|---|---|---|---|---|
| mV | mV/s | mA/cm² Stiekelektrode (Variante A) | mA/cm² GE | mA/cm² Stickelektrode (Variante B) Cu | mA/cm² GE | mA/cm² Stickelektrode (Variante C) Cu | mA/cm² GE | mA/cm² Cu-Elektrode (Platte) | mA/cm² GE |
| -800 | 1 | 0,50 | 0,28 | 0.76 | 0,17 | 1,05 | 0,17 | 0,32 | 0,10 |
| | 5 | 0,80 | 0,34 | 0,92 | 0,21 | 1,15 | 0,42 | 0,52 | 0,23 |
| | 10 | 0,91 | 0,50 | 0.99 | 0.27 | 1,13 | 0,60 | 0,52 | 0,23 |
| -900 | 1 | 0,58 | 0,35 | 0.91 | 0.19 | 1.42 | 0,29 | 0,33 | 0,09 |
| | 5 | 0,93 | 0,39 | 1,01 | 0,24 | 1,75 | 0,5 | 0,42 | 0,16 |
| | 10 | 1,20 | 0,52 | 1,18 | 0,26 | 1,82 | 0,62 | 0,49 | 0,16 |
| -1000 | 1 | 0,62 | 0,27 | 1,02 | 0,24 | 1,55 | 0,38 | 0,38 | 0,09 |
| | 5 | 0,97 | 0,41 | 1.13 | 0.29 | 1,98 | 0,56 | 0,46 | 0,14 |
| | 10 | 1,48 | 0,56 | 1,23 | 0.31 | 2,24 | 0,67 | 0,50 | 0,18 |

Bei der gestickten Edelstahlelektrode (Elektrodenvariante A) wird eine im Vergleich zur Cu-Platte um den Faktor 2 erhöhte Stromdichte beobachtet, bei der gestickten Cu- Elektrode wird je nach Bauform (Elektrodenvariante B oder C) trotz der sehr geringen Oberfläche des Drahts eine außerordentlich hohe Stromdichte beobachtet, welche um einen Faktor 2 - 4 über der massiven Cu-Plattenelektrode liegt. Elektrodenvariante B ist insbesondere von Interesse, wenn besonders kostenintensive Materialien z.B. Pt, oder katalytisch wirksame Legierungen sparsam eingesetzt werden müssen.

### Anwendungsbeispiel 2:

Die Experimente wurden bei Raumtemperatur durchgeführt. Die Voltammogramme wurden in einer Durchflusszelle mit paralleler Geometrie durchgeführt. Katolyt und Anolyt werden durch eine Kationtauschermembrane getrennt (Nafion Type). Als Kathode wird zu Vergleichszwecken eine plane Cu-Katode mit 100 cm² Fläche verwendet (Cu-Folie, Merck, Darmstadt, Deutschland), für die Anwendungsbeispiele die durch Stickerei hergestellte dreidimensionale Elektrode mit der selben Fläche (Elektrodenvarianten A bis C, siehe oben). Die Anode besteht aus Edelstahl.

Die Versuchslösung wird als Katolyt verwendet und wird mithilfe einer Schlauchpumpe durch den Kathodenraum gepumpt (Strömungsgeschwindigkeit 150 ml/min). Dies entspricht einer Katolytströmung von rund 0,15 cm s⁻¹. Das Gesamtvolumen der Katolytlösung betrug 800 ml. 400 ml 1 M NaOH wurden zur Füllung des Anodenraums verwendet. Im Katolyt wurde 0,1 M NaOH als Grundelektrolyt verwendet. Die Zellenspannung wird entweder manuell durch Regelung an einer Laborstromversorgung eingestellt oder durch einen Potentiostaten so gesteuert, dass sich ein erwünschtes Katodenpotential einstellt. Das Kathodenpotential wird gegenüber einer (Ag/AgCl, 3M KCl) Referenzelektrode gemessen. Bei manueller Regelung wird ein Potentiometer verwendet (Metrohm pH meter 654, Fa. Metrohm, Herisau, Switzerland) bei Verwendung des Potentiostaten übernimmt dieser die Potentialmessung und Regelung. Durch Messung des Zellenstroms als Funktion des sich schrittweise verändernden Kathodenpotentials wird ein Voltammogram vermessen. Als elektrochemisch reversibles Redoxsystem wird eine 0.005 mol/l 1,2-Dihydroxy- 9,10-anthrachinon-3-sulfonatlösung (Alizarin S) verwendet. Bei beiden Elektrodentypen wird die Zellenstromstärke bei einem Kathodenpotential von -800 mV, -900 mV und -1000 mV ermittelt und in Tabellen 2 und 3 dargestellt. Tabelle 2 zeigt Vergleichsdaten zwischen der Referenzelektrode und der Elektrodenvariante A bei händischer Messung (ohne Potentiostaten) bis ein stabiles Signal erreicht wurde. Tabelle 3 zeigt Untersuchungen an den Elektrodenvarianten B und C mit Potentiostat. Die Daten aus Tabelle 2 entsprechen etwa einer Spannungsvorschubgeschwindigkeit von 5 mV/s.

**Tabelle 2 (GE = Grundelektrolyt)**

| Katodenpotential | Stromdichte | | | |
|---|---|---|---|---|
| mV | mA/cm² Stickelektrode (Variante A) | mA/cm² GE | mA/cm² Cu-Elektrode (Platte) | mA/cm² GE |
| -800 | 0,15 | 0,04 | 0,09 | 0,08 |
| -900 | 0,35 | 0,07 | 0,19 | 0,08 |
| -1000 | 0,44 | 0,11 | 0,23 | 0,09 |

**Tabelle 3. (GE = Grundelektrolyt)**

| Katodenpotential | *v* | | | | |
|---|---|---|---|---|---|
| mV | mV/s | mA/cm² | mA/cm² | mA/cm² | mA/cm² |
| | | Stickelektrode (Variante B) Cu | GE | Stickelektrode (Variante C) Cu | GE |
| -800 | 1 | 0,63 | 0,10 | 0,33 | 0,50 |
| | 5 | 0,69 | 0,25 | 0,42 | 0,47 |
| | 10 | 0,68 | 0,36 | 0,43 | 0,55 |
| -900 | 1 | 0,85 | 0,18 | 0,85 | 0,47 |
| | 5 | 1,05 | 0,30 | 0,96 | 0,43 |
| | 10 | 1,09 | 0,37 | 1,05 | 0,53 |
| -1000 | 1 | 0,93 | 0,23 | 1,45 | 0,59 |
| | 5 | 1,19 | 0,34 | 1,61 | 0,48 |
| | 10 | 1,34 | 0,40 | 1,73 | 0,60 |

Bei der gestickten Edelstahlelektrode (Variante A) wird eine im Vergleich zur Cu- Platte um den Faktor 2 erhöhte Stromdichte beobachtet, bei der der gestickten Cu- Elektrode wird je nach Bauform (Varianten B oder C) trotz der geringen Elektrodenoberfläche des Drahts eine hohe Stromdichte beobachtet, welche um einen Faktor 2 - 4 über der massiven Cu-Plattenelektrode liegt. Die Variante B ist insbesondere von Interesse, wenn besonders kostenintensive Materialien z.B. Pt, oder katalytisch wirksame Legierungen sparsam eingesetzt werden müssen.

## Patentansprüche

1. Elektrode für eine galvanische Zelle, **gekennzeichnet durch** einen elektrisch isolierenden Träger (3), welcher mit einer Stickerei (1, 2) versehen ist, wobei zumindest ein Teil der Oberfläche der Stickerei (1, 2) elektrisch leitend ausgebildet ist, wobei die Stickerei eine dreidimensionale elektrisch leitende Struktur bildet und Fäden der Stickerei in Stickzahl und/oder der Stickdichte variiert sind und/oder unterschiedlich in der Elektrodentiefe verteilt sind,
**dadurch gekennzeichnet, dass**
Teile der Stickerei als Luftstickerei ausgebildet sind.

2. Elektrode nach Anspruch 1, **dadurch gekennzeichnet, dass** der Träger (3) ein Vlies, eine Folie, ein Gewebe, ein Gitter oder eine Mischung daraus aufweist.

3. Elektrode nach Anspruch 1 oder Anspruch 2, **dadurch gekennzeichnet, dass** die Stickerei (1, 2) aus einem zumindest an der Oberfläche elektrisch leitenden Material besteht.

4. Elektrode nach einem der Ansprüche 1 bis 3, **dadurch gekennzeichnet, dass** die Stickerei (1, 2) aus Metallfasern, Folienstreifen, Drähten, Carbonfasern, Fasern - vorzugsweise Kunststofffasern oder Spinnfasern - welche gegebenenfalls eine elektrisch leitende Beschichtung aufweisen, oder einer Kombination daraus besteht.

5. Elektrode nach einem der Ansprüche 1 bis 4, **dadurch gekennzeichnet, dass** der Faden, aus dem die Stickerei (1, 2) ausgeführt ist, auch eine Anschlussfahne (5, 6) für einen Leiter aufweist.

6. Elektrode nach einem der Ansprüche 1 bis 5, **dadurch gekennzeichnet, dass** die Leiterdichte der Elektrode über die Elektrodenlänge, -breite und -tiefe derart verteilt ist, dass im Betriebszustand eine homogene Stromdichtenverteilung erzielbar ist.

7. Elektrode nach einem der Ansprüche 1 bis 6, **dadurch gekennzeichnet, dass** katalytisch wirksame Materialien auf der elektrisch leitenden Oberflächeaufgebraucht sind.

8. Galvanische Zelle, umfassend zwei Elektroden die jeweils in einem Elektrolyten eingebettet sind, **dadurch gekennzeichnet, dass** wenigstens eine Elektrode nach einem der Ansprüche 1 bis 7 ausgebildet ist.

9. Galvanische Zelle nach Anspruch 8, **dadurch gekennzeichnet, dass** die Elektrode in eine elektrochemisch aktive Masse eingebettet ist.

10. Galvanische Zelle nach Anspruch 8 oder Anspruch 9, **dadurch gekennzeichnet, dass** es sich um eine Brennstoffzelle handelt, wobei katalytisch wirksame Materialien auf der elektrisch leitenden Oberfläche der Elektrode aufgebraucht sind.

11. Halbelement, umfassend eine Elektrode die in einem Elektrolyten eingebettet ist, **dadurch gekennzeichnet, dass** wenigstens eine Elektrode nach einem der Ansprüche 1 bis 7 ausgebildet ist.

12. Verfahren zur Herstellung einer Elektrode nach einem der Ansprüche 1 bis 7, **dadurch gekennzeichnet, dass** in einen elektrisch isolierenden Träger (3) eine Stickerei (1,2) eingebracht wird, wobei zumindest ein Teil der Oberfläche der Stickerei elektrisch leitend ausgebildet ist, wobei durch Stickerei eine dreidimensionale elektrisch leitende Struktur gebildet wird und Fäden in Stickzahl und/oder Stickdichte variiert werden und/oder unterschiedlich in der Elektrodentiefe verteilt werden,
**dadurch gekennzeichnet, dass**
der Träger (3) nach dem Aufbringen der Stickerei teilweise wieder entfernt wird.

## Claims

1. An electrode for a galvanic cell, **characterized by** an electrically insulating carrier (3) provided with an embroidery (1, 2), wherein at least a part of the surface of the embroidery (1, 2) is configured to be electrically conductive, wherein the embroidery forms a three-dimensional, electrically conductive structure and filaments of the embroidery are varied in stitching number and/or stitching density and/or are distributed differently in the depth of the electrode, **characterized in that** part of the embroidery is configured as air embroidery.

2. An electrode according to claim 1, **characterized in that** the carrier (3) has a non-woven fabric, a film, a fabric, a mesh or a mixture thereof.

3. An electrode according to claim 1 or claim 2, **characterized in that** the embroidery (1, 2) consists of a material that is electrically conductive at least on the surface thereof.

4. An electrode according to any of claims 1 to 3, **characterized in that** the embroidery (1, 2) consists of metal fibres, film strips, wires, carbon fibres, fibres - preferably plastic fibres or staple fibres - which optionally have an electrically conductive coating, or of a combination thereof.

5. An electrode according to any of claims 1 to 4, **characterized in that** the filament, from which the embroidery (1, 2) is made, also has a connection lug (5, 6) for a conductor.

6. An electrode according to any of claims 1 to 5, **characterized in that** the conductor density of the electrode is distributed over the length, width and depth of the electrode so that there may be achieved a homogenous current density distribution in the operative status.

7. An electrode according to any of claims 1 to 6, **characterized in that** catalytically active materials are attached on the electrically conductive surface.

8. A galvanic cell comprising two electrodes, which are each embedded in an electrolyte, **characterized in that** at least one electrode is configured according to any of claims 1 to 7.

9. A galvanic cell according to claim 8, **characterized in that** the electrode is embedded in an electrochemically active mass.

10. A galvanic cell according to claim 8 or claim 9, **characterized in that** it is a fuel cell, wherein catalytically active materials are attached on the electrically conductive surface of the electrode.

11. A half-element comprising an electrode, which is embedded in an electrolyte, **characterized in that** at least one electrode is configured according to any of claims 1 to 7.

12. A method for the production of an electrode according to any of claims 1 to 7, **characterized in that** an embroidery (1, 2) is introduced into an electrically insulating carrier (3), wherein at least a part of the surface of the embroidery is electrically conductive, wherein a three-dimensional electrically conducting structure is formed via embroidery and filaments are varied in stitching number and/or stitching density and/or distributed differently in the depth of the electrode, **characterized in that** the carrier (3) is removed partially after the embroidery has been attached.

## Revendications

1. Électrode pour une cellule galvanique, **caractérisée par** un support électriquement isolant (3), qui est muni d'une broderie (1, 2), dans laquelle au moins une partie de la surface de la broderie (1, 2) est électriquement conductrice, dans laquelle la broderie forme une structure tridimensionnelle électriquement conductrice et des fils de la broderie varient en nombre de points et/ou en densité de points et/ou sont différemment répartis dans la profondeur de l'électrode, **caractérisée en ce que** des parties de la broderie sont réalisées sous forme de broderie aérienne.

2. Électrode selon la revendication 1, **caractérisée en ce que** le support (3) présente un non-tissé, une feuille, un tissu, un treillis ou un mélange de ceux-là.

3. Électrode selon la revendication 1 ou la revendication 2, **caractérisée en ce que** la broderie (1, 2) se compose d'un matériau électriquement conducteur au moins à la surface.

4. Électrode selon l'une quelconque des revendications 1 à 3, **caractérisée en ce que** la broderie (1, 2) se compose de fibres métalliques, de bandes de feuille, de fils, de fibres de carbone, de fibres - de préférence de fibres synthétiques ou de fibres discontinues - qui présentent éventuellement un revêtement électriquement conducteur, ou d'une combinaison de celles-là.

5. Électrode selon l'une quelconque des revendications 1 à 4, **caractérisée en ce que** le fil, avec lequel la broderie (1, 2) est réalisée, présente aussi une cosse de raccordement (5, 6) pour un conducteur.

6. Électrode selon l'une quelconque des revendications 1 à 5, **caractérisée en ce que** la densité de conducteur de l'électrode est répartie sur la longueur, la largeur et la profondeur de l'électrode, de telle manière que, dans l'état de fonctionnement, une répartition homogène de la densité de courant puisse être réalisée.

7. Électrode selon l'une quelconque des revendications 1 à 6, **caractérisée en ce que** des matériaux à action catalytique sont déposés sur la surface électriquement conductrice.

8. Cellule galvanique, comprenant deux électrodes, qui sont chacune incorporées dans un électrolyte, **caractérisée en ce qu'**au moins une électrode est formée selon l'une quelconque des revendications 1 à 7.

9. Cellule galvanique selon la revendication 8, **caractérisée en ce que** l'électrode est incorporée dans une masse électrochimiquement active.

10. Cellule galvanique selon la revendication 8 ou la revendication 9, **caractérisée en ce qu'**il s'agit d'une pile à combustible, dans laquelle des matériaux à action catalytique sont déposés sur la surface électriquement conductrice de l'électrode.

11. Semi-élément, comprenant une électrode qui est incorporée dans un électrolyte, **caractérisé en ce qu'**au moins une électrode est formée selon l'une quelconque des revendications 1 à 7.

12. Procédé de fabrication d'une électrode selon l'une quelconque des revendications 1 à 7, **caractérisé en ce que** l'on introduit une broderie (1,2) dans un support électriquement isolant (3), dans lequel au moins une partie de la surface de la broderie est électriquement conductrice, dans lequel on forme par broderie une structure tridimensionnelle électriquement conductrice et on fait varier les fils en nombres de points et/ou en densité de points et/ou on les répartit différemment dans la profondeur de l'électrode, **caractérisé en ce que** l'on enlève de nouveau partiellement le support (3) après l'application de la broderie.
